# EUROPÄISCHE PATENTANMELDUNG

(11) **EP 4 548 905 A1**
(43) Veröffentlichungstag der Anmeldung: **07.05.2025**
(21) Anmeldenummer: 24204081.4
(22) Anmeldetag: 02.10.2024
(51) Int. Cl.: A61K 8/02, A61K 8/25, A61K 8/34, A61K 8/365, A61Q 5/06

(54) **ZUSAMMENSETZUNG UMFASSEND KIESELSÄUREN ZUR KOSMETISCHEN BEHANDLUNG KERATINISCHER FASERN**

(30) Priorität: 31.10.2023 DE 102023210769
(71) Anmelder: Henkel AG & Co. KGaA, 40589 Düsseldorf (DE)
(72) Erfinder: Puls, Anna, 21423 Winsen (Luhe) (DE); Noll, Marcus, 25451 Quickborn (DE)

(57) **Zusammenfassung**

Die Erfindung betrifft eine Zusammensetzung zur kosmetischen Behandlung keratinischer Fasern enthaltend eine hydrophob modifizierte Kieselsäure und eine hydrophile Kieselsäure, dadurch gekennzeichnet, dass die hydrophile Kieselsäure eine mittlere Partikelgröße d50 im Bereich von 6 bis 18 µm, gemessen gemäß ISO 13320, aufweist. Die Erfindung betrifft außerdem die Verwendung der Zusammensetzung zur kosmetischen Behandlung keratinischer Fasern, insbesondere zur Volumenvergrößerung einer Frisur aus keratinischen Fasern.

## Beschreibung

### Gebiet der Technik

Die Erfindung betrifft eine Zusammensetzung zur kosmetischen Behandlung keratinischer Fasern, die eine Mischung einer hydrophilen Kieselsäure mit geringer Partikelgröße und einer hydrophob modifizierten Kieselsäure umfasst. Die Zusammensetzung wird insbesondere zur Volumenvergrößerung einer Frisur aus keratinischen Fasern verwendet.

### Stand der Technik

Zusammensetzungen zur kosmetischen Behandlung keratinischer Fasern können unter anderem auf Basis von Kieselsäuren (amorphem Siliziumdioxid) als Bestandteil hergestellt werden. Derartige Zusammensetzungen liegen üblicherweise in Puderform vor. Diese Zusammensetzungen dienen beispielsweise zur Pflege und der ästhetischen Verschönerung keratinischer Fasern. Insbesondere können auf Basis von Kieselsäuren sogenannte Volumenpuder hergestellt werden, die keratinischen Fasern ein erhöhtes Volumen und temporäre Verformbarkeit verleihen.

Ein Nachteil bekannter Zusammensetzungen auf Basis von Kieselsäuren besteht darin, dass sie bei Verwendung auf dem Haar einen stumpfen und rauen haptischen Eindruck auf dem behandelten Haar und ggf. auf den Handflächen der Nutzer erzeugen. Dieser Nachteil äußert sich insbesondere bei langen Haaren. Darüber hinaus erzeugen bekannte Zusammensetzungen gelegentlich eine als unschön und stumpf empfundene Textur der Haare.

Eine weitere Eigenschaft bekannter Zusammensetzungen in Puderform ist deren mehr oder weniger ausgeprägte Staubneigung. Zusammensetzungen, die zu trocken sind und aus zu kleinen Partikeln bestehen, neigen eher dazu, beim Auftragen bzw. bei der Entnahme der Zusammensetzung aus einem Behälter zu stauben. Dies ist nachteilig für die Handhabbarkeit der Zusammensetzung.

Um diese Probleme zu lösen, wurden im Stand der Technik Zusammensetzungen auf Basis von modifizierten Kieselsäuren entwickelt. Beispielsweise offenbart DE 10 2018 130 538 A1 Zusammensetzungen enthaltend eine hydrophob modifizierte Kieselsäure und Partikel umfassend ein Silikonelastomer. Diese Zusammensetzungen ergeben sprühbare Volumenpuder zum Bewirken eines weichen Haargefühls. Nachteilig ist hierbei jedoch die Verwendung des Silikonelastomers als Bestandteil der Zusammensetzung, da generell ein Interesse daran besteht, Zusammensetzungen ohne Silikon bereitzustellen, die aus nachhaltigeren Quellen gewonnen werden können und biologisch besser abbaubar sind.

### Technische Aufgabe

Der Erfindung liegt die Aufgabe zugrunde, ein kosmetisches Mittel zur Behandlung keratinischer Fasern bereitzustellen. Das Mittel soll sich insbesondere zur Volumenvergrößerung einer Frisur aus keratinischen Fasern eignen. Ferner soll das Mittel leicht handhabbar sein und den Fasern eine angenehme, geschmeidige Haptik verleihen. Schließlich soll das Mittel möglichst keine silikonhaltigen bzw. biologisch schwer abbaubaren Bestandteile umfassen.

### Beschreibung der Erfindung

Die Erfindung betrifft eine Zusammensetzung zur kosmetischen Behandlung keratinischer Fasern enthaltend
eine hydrophob modifizierte Kieselsäure und
eine hydrophile Kieselsäure,
dadurch gekennzeichnet, dass die hydrophile Kieselsäure eine mittlere Partikelgröße d50 im Bereich von 6 bis 18 µm, gemessen gemäß ISO 13320, aufweist.

Die Zusammensetzung kann vorzugsweise puderförmig sein.

Die hydrophile Kieselsäure kann vorzugsweise eine im Wesentlichen sphärische Partikelform aufweisen.

Die hydrophile Kieselsäure kann vorzugsweise einen Siebrückstand von weniger als 10 %, gemessen auf einem 45 µm Sieb gemäß ISO 3262-19, aufweisen.

Die hydrophob modifizierte Kieselsäure kann vorzugsweise eine Silazan-modifizierte Kieselsäure sein.

Die Zusammensetzung kann vorzugsweise
2 bis 30 Gew.-% der hydrophob modifizierten Kieselsäure und
2 bis 30 Gew.-% der hydrophilen Kieselsäure umfassen,
jeweils bezogen auf das Gesamtgewicht der Zusammensetzung.

Die Zusammensetzung kann zusätzlich
eine organische Säure,
einen mehrwertigen Alkohol und/oder
ein Konservierungsmittel umfassen.

Die organische Säure kann vorzugsweise eine Carbonsäure sein, die eine bis drei Carboxylgruppen und 2 bis 10 Kohlenstoffatome aufweist, der mehrwertige Alkohol kann vorzugsweise 2 bis 6 Kohlenstoffatome und 2 bis 3 OH-Gruppen aufweisen und/oder das Konservierungsmittel kann vorzugsweise Natriumbenzoat umfassen.

Die Erfindung betrifft außerdem eine Verwendung der oben beschriebenen Zusammensetzung zur kosmetischen Behandlung keratinischer Fasern.

Die Zusammensetzung wird vorzugsweise zur Volumenvergrößerung einer Frisur aus keratinischen Fasern und/oder zur Verbesserung der haptischen Eigenschaften von keratinischen Fasern eingesetzt.

### Vorteilhafte Wirkungen der Erfindung

Die Erfindung stellt eine Zusammensetzung zur Behandlung keratinischer Fasern bereit, mit der sich insbesondere das Volumen einer Frisur aus keratinischen Fasern vergrößern lässt. Durch die erfindungsgemäße Kombination einer hydrophob modifizierten Kieselsäure mit einer hydrophilen Kieselsäure mit geringer Partikelgröße kann die Zusammensetzung in einer leicht handhabbaren Puderform bereitgestellt werden, die sich insbesondere durch eine geringe Staubentwicklung auszeichnet. Ferner verleiht die Zusammensetzung nicht nur den keratinischen Fasern eine angenehme Haptik, sondern hinterlässt auch ein angenehmes Gefühl auf den Händen der Nutzer. Gemäß der Erfindung können alle diese Eigenschaften erzielt werden, ohne dass die Zusammensetzung ein aus dem Stand der Technik bekanntes Silikonelastomer umfassen muss. Die erfindungsgemäße Zusammensetzung weist somit die gleichen vorteilhaften Eigenschaften auf wie eine aus dem Stand der Technik bekannte Silikonelastomer-haltige Zusammensetzung oder übertrifft diese sogar. Die Erfindung verzichtet dabei auf silikonhaltige und schlecht biologisch abbaubare Bestandteile.

### Beschreibung der Ausführungsformen

Im Folgenden werden Ausführungsformen der vorliegenden Erfindung im Einzelnen beschrieben. Die vorliegende Erfindung ist jedoch nicht auf die folgenden Ausführungsformen beschränkt.

Die Zusammensetzung umfasst als wesentliche Bestandteile eine hydrophobmodifizierte und eine hydrophile Kieselsäure. Als Kieselsäure wird im Rahmen der Erfindung amorphes Siliziumdioxid bezeichnet. Die Kieselsäuren können beispielsweise durch Fällung aus einer silikathaltigen Lösung (Fällungskieselsäure) oder durch Pyrolyse siliziumhaltiger Verbindungen, insbesondere von Siliziumchlorid, (pyrogene Kieselsäure) hergestellt werden.

Die Kombination dieser Bestandteile ergibt eine Zusammensetzung, die für die kosmetische Behandlung keratinischer Fasern geeignet ist. Als kosmetische Behandlung keratinischer Fasern werden im Rahmen der Erfindung insbesondere die Pflege, Formgebung (insbesondere die temporäre Formgebung) und das Erzeugen von Volumen in Frisuren keratinischer Fasern bezeichnet. Als keratinische Fasern werden im Rahmen der Erfindung insbesondere Pelze, Wolle, Federn und menschliche Haare bezeichnet. Insbesondere eignet sich die Zusammensetzung zur kosmetischen Behandlung menschlicher oder tierischer Haare, ganz besonders zur Behandlung menschlichen Haupthaars.

Die hydrophile Kieselsäure zeichnet sich dadurch aus, dass sie in reinem Wasser vollständig benetzt werden kann. Die hydrophile Kieselsäure kann beispielsweise eine Fällungskieselsäure oder eine pyrogene Kieselsäure sein. Vorzugsweise handelt es sich bei der hydrophilen Kieselsäure um eine Fällungskieselsäure. Als hydrophile Kieselsäure kann auch eine Mischung hydrophiler Kieselsäuren eingesetzt werden. Vorzugsweise umfasst die hydrophile Kieselsäure keinerlei Modifikationen mit einer organischen Komponente.

Vorzugsweise handelt es sich um eine amorphe Kieselsäure, die keine kristallinen Bestandteile enthält. Der kristalline Anteil kann über Röntgenbeugung bestimmt werden und liegt vorzugsweise unterhalb einer Nachweisgrenze von 0,1 Gew.-%.

Von entscheidender Bedeutung für die Erfindung ist, dass die hydrophile Kieselsäure eine mittlere Partikelgröße d50 im Bereich von 6 bis 18 µm aufweist. Die mittlere Partikelgröße d50 wird gemäß ISO 13320 in der am Prioritätstag der Erfindung gültigen aktuellen Fassung gemessen. Insbesondere kann die mittlere Partikelgröße d50 mittels Laserbeugung bestimmt werden.

Mit einer mittleren Partikelgröße d50 im erfindungsgemäßen Bereich werden die oben beschriebenen vorteilhaften Eigenschaften der Zusammensetzung erzielt. Insbesondere verleiht die Verwendung einer hydrophilen Kieselsäure mit der angegebenen Partikelgröße der Zusammensetzung die beschriebenen angenehmen haptischen Eigenschaften, insbesondere nach Trocknung der etwaigen flüssigen Bestandteile der Zusammensetzung. Gleichzeitig behält die Zusammensetzung ihre Volumen-vergrößernde Wirkung auf keratinische Fasern. Außerdem gewährleistet die angegebene Partikelgröße eine geringe Staubbildung im Falle einer puderförmigen Zusammensetzung. Darüber hinaus sind die Partikel nicht so groß, dass nach einer Anwendung auf den keratinischen Fasern sichtbar wären. Durch die Verwendung der hydrophilen Kieselsäure kann auf die Zugabe von Silikonelastomeren in der Zusammensetzung verzichtet werden. Diese Vorteile können konnten bislang mit einer hydrophilen Kieselsäure mit abweichender Partikelgröße d50 nicht erzielt werden.

Die hydrophile Kieselsäure weist vorzugsweise eine mittlere Partikelgröße d50 im Bereich von 7 bis 14 µm, bevorzugt im Bereich von 8 bis 12 µm, am meisten bevorzugt ein mittlere Partikelgröße d50 im Bereich von 9 bis 10 µm auf.

Die hydrophile Kieselsäure weist vorzugweise eine enge Partikelgrößenverteilung auf. Die Partikelgrößenverteilung kann anhand der Volumensummenverteilung der gemäß ISO 13320 gemessenen Partikelgrößen bestimmt werden. In einer Ausführungsform weist die Kieselsäure eine Partikelgrößenverteilung auf, die durch die Parameter d90 und d10 gekennzeichnet ist, wobei d90 den maximalen Durchmesser von 90 % der gemessenen Partikel und d10 den maximalen Durchmesser von 10 % der gemessenen Partikel angibt. Vorzugsweise hat d90 einen Wert von 60 µm oder weniger, besonders bevorzugt 50 µm oder weniger, am meisten bevorzugt 40 µm oder weniger. Vorzugsweise hat d10 einen Wert von 5 µm oder weniger, besonders bevorzugt 3 µm oder weniger, am meisten bevorzugt 1 µm oder weniger. Vorzugsweise hat d90 einen Wert von 60 µm oder weniger und d10 einen Wert von 5 µm oder weniger, besonders bevorzugt hat d90 einen Wert von 50 µm oder weniger und d10 einen Wert von 3 µm oder weniger, am meisten bevorzugt hat d90 einen Wert von 40 µm oder weniger und d10 einen Wert von 1 µm oder weniger.

Die hydrophile Kieselsäure weist vorzugsweise einen geringen Koagulatanteil, also einen geringen Anteil koagulierter Partikel auf. Der Koagulatanteil kann durch Sieben anhand des Siebrückstands der Kieselsäure bestimmt werden. Vorzugsweise wird der Siebrückstand auf einem 45 µm Sieb gemäß ISO 3262-19 in der am Prioritätstag der Erfindung gültigen aktuellen Fassung bestimmt. Die hydrophile Kieselsäure weist vorzugsweise einen Siebrückstand von 10 % oder weniger, besonders bevorzugt 5 % oder weniger, am meisten bevorzugt 1 % oder weniger auf, jeweils gemessen auf einem 45 µm Sieb gemäß ISO 3262-19.

Die hydrophile Kieselsäure besteht vorzugsweise aus Partikeln, die eine im Wesentlichen sphärische Partikelform aufweisen. Die Partikelform kann anhand des folgenden Verfahrens bestimmt werden. Von einer Probe der Kieselsäure wird mittels Rasterelektronenmikroskopie ein Bild aufgenommen. Anhand des Bildes werden von den einzelnen Partikeln jeweils die Querschnittsfläche A und der Umfang U ermittelt. Der Grad der sphärischen Partikelform (Sphärizität) der einzelnen Partikel wird anhand des Parameters S quantifiziert, wobei S aus der Gleichung S = U² / (4 π A) berechnet wird. Für eine ideale Kugelform ist S = 1. Je unregelmäßiger der Partikel geformt ist und je stärker die Partikelform von der idealen Kugelform abweicht, desto kleiner ist S. Anhand des Durchschnitts der Sphärizität der einzelnen Partikel wird die Sphärizität der gesamten Probe bestimmt. In einer Ausführungsform weist die hydrophile Kieselsäure eine anhand dieses Verfahrens bestimmte Sphärizität von 0,80 oder mehr, bevorzugt 0,85 oder mehr, besonders bevorzugt 0,90 oder mehr, am meisten bevorzugt 0,95 oder mehr auf.

Die hydrophile Kieselsäure weist vorzugsweise eine BET-Oberfläche von 24 m²/g oder weniger, besonders bevorzugt 18 m²/g oder weniger, am meisten bevorzugt 12 m²/g oder weniger auf. Die BET-Oberfläche kann gemäß DIN 66131 in der am Prioritätstag der Anmeldung gültigen aktuellen Fassung bestimmt werden.

Geeignete hydrophile Kieselsäuren sind beispielsweise unter dem Handelsnamen Spherilex^{®} der Firma Evonik erhältlich.

Die Zusammensetzung enthält vorzugsweise 2 bis 30 Gew.-%, bevorzugt 3 bis 20 Gew.-%, besonders bevorzugt 5 bis 15 Gew.-%, am meisten bevorzugt 6 bis 12 Gew.-% der hydrophilen Kieselsäure, bezogen auf das Gesamtgewicht der Zusammensetzung.

Vorzugsweise umfasst die Zusammensetzung mindestens eine der als bevorzugt beschriebenen hydrophilen Kieselsäuren, bevorzugt in der oben angegebenen Menge. Der insgesamte Anteil hydrophiler Kieselsäuren ist in diesem Fall vorzugsweise auf bis zu 30 Gew.-%, bevorzugt bis zu 20 Gew.-%, besonders bevorzugt bis zu 15 Gew.-%, am meisten bevorzugt bis zu 12 Gew.-% eingeschränkt.

Die hydrophobe Kieselsäure zeichnet sich durch eine hydrophobe Oberfläche aus, die von reinem Wasser nicht vollständig benetzt wird. Die hydrophob modifizierte Kieselsäure kann beispielsweise durch Modifikation einer Fällungskieselsäure oder einer pyrogenen Kieselsäure erhalten werden. Besonders bevorzugt ist die hydrophob modifizierte Kieselsäure eine pyrogene Kieselsäure. Als hydrophob modifizierte Kieselsäure kann auch eine Mischung hydrophob modifizierter Kieselsäuren eingesetzt werden.

Die hydrophob modifizierte Kieselsäure kann beispielsweise eine hydrophobe organische Beschichtung aufweisen. Beispielsweise kann die Kieselsäure mit einem Wachs beschichtet sein.

Die hydrophob modifizierte Kieselsäure kann auch durch Modifikation mit einer Siloxan-, Silan- oder Silazanverbindung erhalten werden (Siloxan-, Silan- oder Silazan-modifizierte Kieselsäure). Derartig modifizierte Kieselsäuren lassen sich besonders gut in der Zusammensetzung verwenden und führen in Kombination mit der beschriebenen hydrophilen Kieselsäure zu den oben genannten Vorteilen. Zur Modifizierung der Kieselsäuren eignen sich besonders Alkylsiloxane, wie beispielsweise Hexamethyldisiloxan, Organosilanverbindungen, wie beispielsweise Dimethylsilan oder Trimethylsilan, oder Silazanverbindungen, wie beispielsweise Hexamethyldisilazan (HDMS). Davon ausgeschlossen sind jedoch Polyorganosiloxan-modifizierte Kieselsäuren.

Besonders bevorzugt handelt es sich bei der hydrophob modifizierten Kieselsäure um eine Silazan-modifizierte Kieselsäure, insbesondere eine mit HDMS modifizierte Kieselsäure. In einer Ausführungsform handelt es sich bei der hydrophob modifizierten Kieselsäure um eine Silazan-modifizierte pyrogene Kieselsäure, insbesondere eine mit HDMS modifizierte pyrogene Kieselsäure.

Vorzugsweise hat die hydrophob modifizierte Kieselsäure einen Kohlenstoffgehalt von 0,1 bis 15 Gew.-%, bevorzugt 1 bis 10 Gew.-%, besonders bevorzugt 2 bis 5 Gew.-%, bezogen auf das Gesamtgewicht der hydrophob modifizierten Kieselsäure.

Die hydrophob modifizierte Kieselsäure weist vorzugsweise eine BET-Oberfläche von 100 m²/g oder mehr auf. Besonders bevorzugt liegt die BET-Oberfläche im Bereich von 100 bis 300 m²/g, am meisten bevorzugt im Bereich von 150 bis 250 m²/g. Die BET-Oberfläche kann gemäß DIN 66131 in der am Prioritätstag der Anmeldung gültigen aktuellen Fassung bestimmt werden.

Geeignete hydrophob modifizierte Kieselsäuren sind beispielsweise unter dem Handelsnamen Aerosil^{®} der Firma Evonik erhältlich.

Die Zusammensetzung enthält vorzugsweise 2 bis 30 Gew.-%, bevorzugt 3 bis 20 Gew.-%, besonders bevorzugt 5 bis 15 Gew.-%, am meisten bevorzugt 6 bis 12 Gew.-% der hydrophob modifizierten Kieselsäure, bezogen auf das Gesamtgewicht der Zusammensetzung.

Vorzugsweise umfasst die Zusammensetzung mindestens eine der als bevorzugt beschriebenen hydrophob modifizierten Kieselsäuren, bevorzugt in der oben angegebenen Menge. Der insgesamte Anteil hydrophob modifizierter Kieselsäuren ist in diesem Fall vorzugsweise auf bis zu 30 Gew.-%, bevorzugt bis zu 20 Gew.- %, besonders bevorzugt bis zu 15 Gew.-%, am meisten bevorzugt bis zu 12 Gew.- % eingeschränkt.

Die Zusammensetzung umfasst vorzugsweise 2 bis 30 Gew.-%, bevorzugt 3 bis 20 Gew.-%, besonders bevorzugt 5 bis 15 Gew.-%, am meisten bevorzugt 6 bis 12 Gew.-% hydrophob modifizierte Kieselsäure und 2 bis 30 Gew.-%, bevorzugt 3 bis 20 Gew.-%, besonders bevorzugt 5 bis 15 Gew.-%, am meisten bevorzugt 6 bis 12 Gew.-% der hydrophilen Kieselsäure, jeweils bezogen auf das Gesamtgewicht der Zusammensetzung.

Die Zusammensetzung kann weitere Bestandteile umfassen. Insbesondere kann die Zusammensetzung zusätzlich eine organische Säure, einen mehrwertigen Alkohol und/oder ein Konservierungsmittel umfassen.

Die organische Säure umfasst vorzugsweise eine ein-, zwei- oder dreiwertige organische Säure, bevorzugt eine ein-, zwei- oder dreiwertige gesättigte Carbonsäure. Es können auch Mischungen organischer Säuren eingesetzt werden. Besonders bevorzugt umfasst die organische Säure eine Carbonsäure, die eine bis drei Carboxylgruppen und 2 bis 10 Kohlenstoffatome aufweist. Am meisten bevorzugt umfasst die organische Säure eine Carbonsäure, die zwei oder drei Carboxylgruppen und 2 bis 10 Kohlenstoffatome aufweist. In einer Ausführungsform umfasst die organische Säure Zitronensäure.

Die Zusammensetzung umfasst vorzugsweise 0,05 bis 0,5 Gew.-%, bevorzugt 0,10 bis 0,25 Gew.-%, besonders bevorzugt 0,10 bis 0,10 Gew.-% organische Säure, jeweils bezogen auf das Gesamtgewicht der Zusammensetzung. Der insgesamte Anteil an organischen Säuren ist auch bei Verwendung einer der als bevorzugt beschriebenen organischen Säuren vorzugsweise auf bis zu 0,5 Gew.- %, bevorzugt bis zu 0,25 Gew.-%, am meisten bevorzugt bis zu 0,10 Gew.-% eingeschränkt.

Der mehrwertige Alkohol kann erfindungsgemäß als wasserlösliches Pflegemittel dienen. Ferner lässt sich durch Zugabe eines mehrwertigen Alkohols auch die Applizierbarkeit der Zusammensetzung verbessern. Vorzugsweise weist der mehrwertige Alkohol 2 bis 6 Kohlenstoffatome und 2 bis 3 OH-Gruppen. Es können auch Mischungen mehrwertiger Alkohole eingesetzt werden. Der mehrwertige Alkohol kann beispielsweise aus der Gruppe bestehend aus Ethylenglycol, 1,2-Propylenglycol, 2-Methyl-1,3-propandiol, Glycerin, 1,2-Butylenglycol, 1,3-Butylenglycol, 1,4-Butylenglycol, 1,2-Pentandiol und 1,5-Pentandiol ausgewählt sein. Besonders bevorzugt ist die Verwendung von Glycerin, 1,2-Propandiol.

Die Zusammensetzung umfasst vorzugsweise 2 bis 18 Gew.-%, bevorzugt 5 bis 15 Gew.-%, besonders bevorzugt 8 bis 12 Gew.-% mehrwertigen Alkohol, jeweils bezogen auf das Gesamtgewicht der Zusammensetzung. Der insgesamte Anteil an mehrwertigen Alkoholen ist auch bei Verwendung eines der als bevorzugt beschriebenen mehrwertigen Alkoholen vorzugsweise auf bis zu 18 Gew.-%, bevorzugt bis zu 15 Gew.-%, am meisten bevorzugt bis zu 12 Gew.-% eingeschränkt.

Das Konservierungsmittel umfasst vorzugsweise mindestens eine bakteriostatische oder fungistatische Verbindung. Es können auch Mischungen unterschiedlicher Konservierungsmittel eingesetzt werden. Vorzugsweise umfasst das Konservierungsmittel Natriumbenzoat. Die Zusammensetzung umfasst vorzugsweise 0,1 bis 1 Gew.-% Konservierungsmittel, bezogen auf das Gesamtgewicht der Zusammensetzung. Der insgesamte Anteil an Konservierungsmitteln ist auch bei Verwendung eines der als bevorzugt beschriebenen Konservierungsmittel vorzugsweise auf bis zu 1 Gew.-% eingeschränkt.

Ferner kann die Zusammensetzung weitere Hilfsstoffe umfassen, die typischerweise in Zusammensetzung zur Behandlung keratinischer Fasern, insbesondere in Volumenpudern, enthalten sind.

Beispielsweise kann die Zusammensetzung einen Duftstoff (Parfüm) umfassen. Der Duftstoff kann eine Mischung unterschiedlicher Duftstoffe enthalten. Die Menge an Duftstoff ist vorzugsweise auf 0,01 bis 1 Gew.-% eingeschränkt, bezogen auf das Gesamtgewicht der Zusammensetzung.

Ferner kann die Zusammensetzung Pflanzenextrakte umfassen. Beispielsweise kann die Zusammensetzung einen Kokosextrakt enthalten. Die Pflanzenextrakte sind vorzugsweise auf wässriger Basis und können geringe Mengen an Alkohol enthalten. Die Menge an Pflanzenextrakt ist vorzugsweise auf 0,01 bis 5 Gew.-%, bevorzugt 0,1 bis 2 Gew.-% eingeschränkt, bezogen auf das Gesamtgewicht der Zusammensetzung.

Die Zusammensetzung umfasst vorzugsweise keine oder nur geringe Mengen an Silikonelastomeren. Als Silikonelastomere werden im Rahmen der Erfindung insbesondere Polyorganosiloxane bezeichnet, insbesondere vernetzte Polyorganosiloxane, insbesondere Polyorganosiloxane mit mehr als drei Siloxaneinheiten. Vorzugsweise ist die Menge an Silikonelastomeren und/oder die an Menge an Polyorganosiloxanen mit mehr als drei Siloxaneinheiten auf 5 Gew.- % oder weniger, bevorzugt 1 Gew.-% oder weniger, am meisten bevorzugt 0,1 Gew.-% oder weniger eingeschränkt, jeweils bezogen auf das Gesamtgewicht der Zusammensetzung.

Die Zusammensetzung umfasst vorzugsweise Wasser, bevorzugt in einer Menge von 40 bis 96 Gew.-%, besonders bevorzugt 60 bis 94 Gew.-%, weiter bevorzugt 65 bis 90 Gew.-%, am meisten bevorzugt 70 bis 88 Gew.-%, jeweils bezogen auf das Gesamtgewicht der Zusammensetzung.

Die Zusammensetzung ist vorzugsweise puderförmig. Vorzugsweise hat eine puderförmige Zusammensetzung einen durch Sieben bestimmten Teilchendurchmesser von 0,5 mm oder weniger, bevorzugt 0,1 mm oder weniger.

In einer Ausführungsform umfasst die Zusammensetzung eine Silazan-modifizierte Kieselsäure, eine hydrophile Kieselsäure, die eine mittlere Partikelgröße d50 im Bereich von 6 bis 18 µm aufweist, einen mehrwertigen Alkohol, ein Konservierungsmittel und eine organische Säure.

In einer Ausführungsform umfasst die Zusammensetzung eine Silazan-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm aufweist, einen mehrwertigen Alkohol, ein Konservierungsmittel und eine organische Säure.

In einer Ausführungsform umfasst die Zusammensetzung eine HDMS-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm aufweist, einen mehrwertigen Alkohol, ein Konservierungsmittel und eine organische Säure.

In einer Ausführungsform umfasst die Zusammensetzung eine Silazan-modifizierte Kieselsäure, eine hydrophile Kieselsäure, die eine mittlere Partikelgröße d50 im Bereich von 6 bis 18 µm, eine mittlere Partikelgröße d90 von 60 µm oder weniger und eine mittlere Partikelgröße d10 von 5 µm oder weniger aufweist, einen mehrwertigen Alkohol, ein Konservierungsmittel und eine organische Säure.

In einer Ausführungsform umfasst die Zusammensetzung eine Silazan-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm, eine mittlere Partikelgröße d90 von 60 µm oder weniger und eine mittlere Partikelgröße d10 von 5 µm oder weniger aufweist, einen mehrwertigen Alkohol, ein Konservierungsmittel und eine organische Säure.

In einer Ausführungsform umfasst die Zusammensetzung eine HDMS-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm, eine mittlere Partikelgröße d90 von 60 µm oder weniger und eine mittlere Partikelgröße d10 von 5 µm oder weniger aufweist, einen mehrwertigen Alkohol, ein Konservierungsmittel und eine organische Säure.

In einer Ausführungsform umfasst die Zusammensetzung eine Silazan-modifizierte Kieselsäure, eine hydrophile Kieselsäure, die eine mittlere Partikelgröße d50 im Bereich von 6 bis 18 µm und eine Sphärizität von 0,90 oder mehr aufweist, einen mehrwertigen Alkohol, ein Konservierungsmittel und eine organische Säure.

In einer Ausführungsform umfasst die Zusammensetzung eine Silazan-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm und eine Sphärizität von 0,90 oder mehr aufweist, einen mehrwertigen Alkohol, ein Konservierungsmittel und eine organische Säure.

In einer Ausführungsform umfasst die Zusammensetzung eine HDMS-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm und eine Sphärizität von 0,90 oder mehr aufweist, einen mehrwertigen Alkohol, ein Konservierungsmittel und eine organische Säure.

In einer Ausführungsform umfasst die Zusammensetzung eine Silazan-modifizierte Kieselsäure, eine hydrophile Kieselsäure, die eine mittlere Partikelgröße d50 im Bereich von 6 bis 18 µm und eine BET-Oberfläche von 24 m²/g oder weniger aufweist, einen mehrwertigen Alkohol, ein Konservierungsmittel und eine organische Säure.

In einer Ausführungsform umfasst die Zusammensetzung eine Silazan-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm und eine BET-Oberfläche von 24 m²/g oder weniger aufweist, einen mehrwertigen Alkohol, ein Konservierungsmittel und eine organische Säure.

In einer Ausführungsform umfasst die Zusammensetzung eine HDMS-modifizierte Kieselsäure mit einer BET-Oberfläche im Bereich von 100 bis 300 m²/g, eine hydrophile Kieselsäure, die eine mittlere Partikelgröße d50 im Bereich von 6 bis 18 µm und eine BET-Oberfläche von 24 m²/g oder weniger aufweist, einen mehrwertigen Alkohol, ein Konservierungsmittel und eine organische Säure.

In einer Ausführungsform umfasst die Zusammensetzung
5 bis 20 Gew.-% mehrwertigen Alkohol,
0,1 bis 1 Gew.-% Konservierungsmittel,
0,05 bis 0,5 Gew.-% organische Säure,
5 bis 15 Gew.-% hydrophile Kieselsäure mit einer mittleren Partikelgröße d50 im Bereich von 6 bis 18 µm,
5 bis 15 Gew.-% Silazan-modifizierte Kieselsäure,
0,1 bis 1 Gew.-% Duftstoffe, und
60 bis 85 Gew.-% Wasser,
jeweils bezogen auf das Gesamtgewicht der Zusammensetzung.

In einer Ausführungsform umfasst die Zusammensetzung
5 bis 20 Gew.-% Glycerin,
0,1 bis 1 Gew.-% Konservierungsmittel,
0,05 bis 0,5 Gew.-% Zitronensäure,
5 bis 15 Gew.-% hydrophile Fällungskieselsäure mit einer mittleren Partikelgröße d50 im Bereich von 6 bis 18 µm,
5 bis 15 Gew.-% HDMS-modifizierte pyrogene Kieselsäure,
0,1 bis 1 Gew.-% Duftstoffe, und
60 bis 85 Gew.-% Wasser,
jeweils bezogen auf das Gesamtgewicht der Zusammensetzung.

Gemäß der Erfindung wird die Zusammensetzung zur kosmetischen Behandlung keratinischer Fasern verwendet. Insbesondere wird die Zusammensetzung zur kosmetischen Behandlung menschlicher Haare verwendet. Vorzugsweise wird die Zusammensetzung zur Formgebung keratinischer Fasern, zum Texturieren keratinischer Fasern, zur Volumenvergrößerung einer Frisur aus keratinischen Fasern oder zur Verbesserung der Oberflächeneigenschaften, insbesondere der haptischen Eigenschaften keratinischer Fasern verwendet. Beispielsweise kann die Zusammensetzung verwendet werden, um keratinischen Fasern, insbesondere menschlichem Haar, eine angenehme, geschmeidige Haptik zu verleihen. In einer bevorzugten Ausführungsform wird die Zusammensetzung zur Volumenvergrößerung einer Frisur aus keratinischen Fasern, insbesondere einer menschlichen Haarfrisur verwendet.

Die Zusammensetzung wird vorzugsweise in Puderform verwendet.

Die Zusammensetzung kann bei der Verwendung beispielsweise mit den Händen oder durch Sprühen auf die keratinischen Fasern aufgetragen werden. Besonders bevorzugt wird die Zusammensetzung durch Sprühen aufgetragen.

In einer Ausführungsform umfasst die Erfindung auch einen Sprühapplikator, der die Zusammensetzung enthält. Der Sprühapplikator verfügt über einen Behälter zur Aufnahme der Zusammensetzung und einen Mechanismus zum Versprühen der Zusammensetzung. Vorzugsweise enthält der Sprühapplikator die Zusammensetzung in Puderform. Vorzugsweise umfasst der Sprühapplikator einen Pumpmechanismus, der durch Erzeugen eines Luftstroms die Zusammensetzung aufwirbelt und versprüht. Auf den Einsatz eines Treibmittels kann somit verzichtet werden.

### Beispiele

Nachfolgend wird die vorliegende Erfindung anhand von Beispielen genauer beschrieben. Die vorliegende Erfindung ist jedoch nicht auf die folgenden Beispiele beschränkt.

Eine erfindungsgemäße Zusammensetzung wurde gemäß der folgenden Rezeptur durch Mischen der angegebenen Bestandteile hergestellt.

| **Bestandteil** | **Gew.-%** |
|---|---|
| Glycerin (99,5 %) | 10,0 |
| Kokosextrakt | 1,0 |
| Meersalz | 0,2 |
| Natriumbenzoat | 0,4 |
| Zitronensäure Monohydrat | 0,12 |
| Aerosil^{®} R 812 S (HDMS-modifizierte pyrogene Kieselsäure) | 7,5 |
| Spherilex^{®} 10 PC (hydrophile Kieselsäure mit d50 = 8,0 bis 12,0 µm) | 7,5 |
| Duftstoffzusammensetzung | 0,3 |
| Wasser | 72,98 |

Es ergab sich eine puderförmige Zusammensetzung, die sich per Hand ohne Staubentwicklung in Haare einmassieren ließ. Anschließend konnte das Haar in die gewünschte Form gebracht werden. Die Zusammensetzung hat eine Volumen-vergrößernde Wirkung auf das Haar und hinterließ einen angenehmen haptischen Eindruck auf Haaren und Händen.

## Patentansprüche

1. Zusammensetzung zur kosmetischen Behandlung keratinischer Fasern enthaltend
eine hydrophob modifizierte Kieselsäure und
eine hydrophile Kieselsäure,
**dadurch gekennzeichnet, dass** die hydrophile Kieselsäure eine mittlere Partikelgröße d50 im Bereich von 6 bis 18 µm, gemessen gemäß ISO 13320, aufweist.

2. Zusammensetzung gemäß Anspruch 1, **dadurch gekennzeichnet, dass** die Zusammensetzung puderförmig ist.

3. Zusammensetzung gemäß Anspruch 1 oder 2, **dadurch gekennzeichnet, dass** die hydrophile Kieselsäure eine im Wesentlichen sphärische Partikelform aufweist.

4. Zusammensetzung gemäß einem der Ansprüche 1 bis 3, **dadurch gekennzeichnet, dass** die hydrophile Kieselsäure einen Siebrückstand von weniger als 10 %, gemessen auf einem 45 µm Sieb gemäß ISO 3262-19, aufweist.

5. Zusammensetzung gemäß einem der Ansprüche 1 bis 4, **dadurch gekennzeichnet, dass** die hydrophob modifizierte Kieselsäure eine Silazan-modifizierte Kieselsäure ist.

6. Zusammensetzung gemäß einem der Ansprüche 1 bis 5, **dadurch gekennzeichnet, dass** die Zusammensetzung
2 bis 30 Gew.-% der hydrophob modifizierten Kieselsäure und
2 bis 30 Gew.-% der hydrophilen Kieselsäure umfasst,
jeweils bezogen auf das Gesamtgewicht der Zusammensetzung.

7. Zusammensetzung gemäß einem der Ansprüche 1 bis 6, **dadurch gekennzeichnet, dass** die Zusammensetzung zusätzlich
eine organische Säure,
einen mehrwertigen Alkohol und/oder
ein Konservierungsmittel umfasst.

8. Zusammensetzung gemäß Anspruch 7, **dadurch gekennzeichnet, dass**
die organische Säure eine Carbonsäure ist, die eine bis drei Carboxylgruppen und 2 bis 10 Kohlenstoffatome aufweist,
der mehrwertige Alkohol 2 bis 6 Kohlenstoffatome und 2 bis 3 OH-Gruppen aufweist und/oder
das Konservierungsmittel Natriumbenzoat umfasst.

9. Verwendung einer Zusammensetzung gemäß einem der Ansprüche 1 bis 8 zur kosmetischen Behandlung keratinischer Fasern.

10. Verwendung gemäß Anspruch 9, **dadurch gekennzeichnet, dass** die Zusammensetzung zur Volumenvergrößerung einer Frisur aus keratinischen Fasern und/oder zur Verbesserung der haptischen Eigenschaften von keratinischen Fasern eingesetzt wird.
